# EUROPEAN PATENT APPLICATION

(11) **EP 4 714 438 A1**
(43) Date of publication of application: **25.03.2026**
(21) Application number: 24201675.6
(22) Date of filing: 20.09.2024
(51) Int. Cl.: A61K 31/196, A61K 31/277, A61K 31/4196, A61K 31/42, A61K 31/437, A61K 31/44, A61K 31/47, A61K 31/505, A61P 35/00, A61P 35/02

(54) **USE OF DHODH INHIBITORS IN TREATMENT OF A SMARCB1 MUTANT ASSOCIATED CANCER**

(71) Applicant: UMC Utrecht Holding B.V., 3584 CS Utrecht (NL); Universiteit Utrecht Holding B.V., 3584 CS Utrecht (NL)
(72) Inventor: Drost, Jarno, 3584 CS Utrecht (NL); Kes, Marjolein, 3584 CS Utrecht (NL); Berkers, Celia, 3584 CS Utrecht (NL)
(74) Representative: Pot, Emil

(57) **Abstract**

The invention covers a novel use of DHODH inhibitors for the treatment of cancer in a patient. In particular, there are provided agents and methods for use in the treatment of cancer wherein the level of functional activity of SMARCB1 in the cancer cells is used to determine whether the patient would benefit from treatment with an inhibitor of the DHODH enzyme.

## Description

### Field of the invention

The present invention relates to novel and less toxic therapies and methods for the treatment of cancer in a patient. In particular, there are provided agents and methods for use in the treatment of cancer wherein the level of functional activity of SMARCB1 in the cancer cells is used to determine whether the patient would benefit from treatment with an inhibitor of the DHODH enzyme.

### Background

Malignant rhabdoid tumors (MRTs) are lethal cancers that predominantly strike young children, mostly infants. The vast majority of MRTs contain bi-allelic inactivating mutations in the *SMARCB1* gene (see Roberts & Biegel, 2009, Cancer Biol. Ther. 8(5), 412-416).

MRTs are highly malignant neoplasms that typically arise in infancy and early childhood. The tumors develop in the brain and spinal cord [referred to as atypical teratoid/rhabdoid tumor (AT/RT)], kidney and/or soft tissues (collectively named extracranial MRT, or ECRT). The histologic appearance of these malignancies can be quite variable. Most tumors contain at least some fields with classic rhabdoid cells, with large nuclei containing a single prominent nucleolus, and cytoplasm with distinct pale eosinophilic inclusions. Tumors demonstrating only classic rhabdoid cells are rare, instead, they often have areas composed of spindled or pleomorphic undifferentiated cells without a rhabdoid phenotype. A classic rhabdoid component may be entirely absent. AT/RTs typically demonstrate a variety of primitive neuroectodermal, epithelial or mesenchymal cells, which underlies the difficulty in distinguishing these tumors from other primitive neuroectodermal tumors or choroid plexus carcinomas. Immunohistochemistry is often used in the differential diagnosis, based on the typical expression of smooth muscle actin, epithelial membrane antigen and vimentin. Lack of expression of the SMARCB1 protein is primarily employed as a specific means of distinguishing rhabdoid tumors from other malignancies with similar histologic features, especially for diagnosis of AT/RT versus primitive neuroectodermal tumors.

Individuals with germline alterations of *SMARCB1* are predisposed to MRT and may present with more than one primary tumor. These children are most often diagnosed within the first year of life and tend to have a worse prognosis. It is not known whether the poor prognosis is related to the presence of a germline mutation in all of their cells, or the fact that they develop multiple and progressive primary tumors that are resistant to therapy.

The name SMARCB1 (SWI/SNF related, Matrix associated, Actin dependent Regulator of Chromatin, subfamily B, member 1) is derived from its role as a core member of the SWI/SNF chromatin remodeling complex. SWI/SNF complexes, also known as BRG1/BRM-associated factor (BAF) complexes, are central regulators of nucleosome remodeling. SWI/SNF complexes are involved in numerous biological processes, including cell cycle regulation and maintenance of genomic stability, and it has been estimated that alterations in SWI/SNF subunits involve over 20% of all cancers.

The SMARCB1 protein (also termed SNF5 or INI1) is highly conserved, as evidenced by an identical amino acid sequence in mice and humans. However, the function of SMARCB1 is poorly understood. There are no SMARCB1 paralogs, and the protein lacks particularly informative protein motifs.

While there are differing chemotherapeutic regimens for cancers with low or absent activity of SMARCB1, such as AT/RT, high-dose methotrexate (MTX) is often part of the treatment regimen, and it is also used in other pediatric indications such as pediatric ALL, non-Hodgkin lymphoma, osteosarcoma, and various brain tumors.

**Despite being part of many pediatric cancer treatment schedules, the use of MTX is associated with severe toxicity and a wide range of side effects.** Patients treated with MTX always receive close monitoring of their health and if required also leucovorin rescue therapy. As expected, daily dosing of MTX in ECRT-bearing mice in vivo is associated with toxicity, indicated by significant weight loss over time in some of the mice. Additionally, it has been shown that patients treated with MTX develop resistance for which the biological mechanisms contributing to the generation of this resistance in AT/RT are unknown. Therefore, there is a great medical need to find a treatment for MRTs, without causing this severe toxicity and wide range of side effects in patients.

As part of the invention hereunder, we identified targetable metabolic vulnerabilities in MRTs, extra-(ECRTs) and intra-cranial (AT/RTs) MRTs. We also demonstrated that, unexpectedly, targeting MRTs with DHODH inhibitors show in vivo/ in vitro efficacy in inhibiting/controlling tumor growth with much less toxicity and side effects than MTX.

DHODH inhibitors appear to be safe in the clinical setting although they have not been shown to be effective for the treatment of cancer (Mullen et al., 2024, eLife 12:RP87292 https://doi.orq/10.7554/eLife.87292.3). For example, BAY-240223423, was discontinued due to lack of benefit for the treatment of myeloid malignancies (https://clinicaltrials.qov/ct2/show/NCT03404726).

In the invention hereunder, we demonstrate that based on the prior art the use of DHODH inhibitors would not be an obvious choice for the treatment of cancer. Nevertheless, our results in vitro in patient-derived organoid (PDO) and in vivo demonstrated a metabolic dependency to DHODH inhibition in rhabdoid tumors, which was not expected.

**The current invention presents an alternative** to decrease the toxicity and side effects caused by the treatment of MRTs, both ECRT and AT/RT, with MTX by using (long-term) DHODH inhibitors (including BAY-2402234). DHODH inhibitors despite having been shown to be safe in various pediatric cancer animal models as well as in a human clinical trial (NCT03404726) for myeloid malignancies, have not yet been shown to be effective for the treatment of cancer.

The present invention seeks to provide novel therapies for the treatment of cancers such as rhabdoid tumors, without the severe toxicity and side effects caused by current treatments, as well as methods of selecting an effective treatment regime in cancer patients.

### Summary of the invention

A first aspect of the invention provides an inhibitor of the human dihydroorotate dehydrogenase (DHODH) enzyme for use in treating cancer in a patient, wherein the cancer is associated with cells in which the functional activity of SMARCB1 (SNF5, INI1) is low or absent.

In an embodiment, the patient treated with the DHODH inhibitor has, or is suspected of having, a cancer selected from the group consisting of malignant rhabdoid tumors, atypical teratoid/rhabdoid tumors, epithelioid sarcomas, synovial sarcomas, undifferentiated sarcomas with or without rhabdoid features, extra skeletal myxoid chondrosarcomas, renal medullary carcinomas, mucinous carcinomas of the pancreas, malignant peripheral nerve sheath tumors, schwannomas, familial and sporadic schwannomatosis, cribriform neuroepithelial tumors, embryonal central nervous system tumors with or without rhabdoid features, choroid plexus carcinomas, teratoma, primitive neuroectodermal tumors, poorly differentiated chordomas, non-Hodgkin lymphoma, and chronic myeloid leukemia.

For example, the patient may have or may be suspected of having an atypical teratoid rhabdoid tumor (AT/RT) and/or a extracranial malignant rhabdoid tumor (ECRT).

Another aspect of the invention is the assessment of the patient to establish that the cancer is associated with cells in which the functional activity of SMARCB1 (SNF5, INI1) is low or absent. By low or absent "functional activity", in this context, we mean reduced or absent expression of SMARCB1 (SNF5, INI1) in tumor cells (for example, relative to internal positive controls in tissue samples, such as normal vessel cells, inflammatory cells, surrounding normal tissue) as assessed at the protein and/or mRNA level (for example, Kohashi K et al., Modern Pathology 2010, 23, 981-990), as well as the presence of chromosomal aberrations or DNA mutations (e.g. deletions, miss-sense, nonsense mutations, and the like) or epigenetic alterations (e.g. DNA methylation) that lead to a reduced or lost SMARCB1 (SNF5, INI1) functioning.

Thus, the low or absent functional activity may manifest itself at the level of the genomic DNA, mRNA, protein and/or activity (i.e. function) of SMARCB1.

It will be appreciated that the assessment step may be performed at any time before or even during treatment of the patient. Preferably, however, the patient is assessed prior to commencement of treatment with the inhibitor.

In one embodiment, the assessment of the patient comprises providing a sample of cells from the patient and measuring the amount of SMARCB1 protein, and/or mRNA encoding the same, in the cells. For example, the assessment of the patient may comprise measuring the amount of SMARCB1 protein in the cells, e.g. by immunohistochemistry, immunofluorescence, Western blot analysis, an immunological assay (e.g., an ELISA or other solid phase-based immunoassay such as SPRIA or amplified ELISA so called IMRAMP), a protein chip assay, surface-enhanced laser desorption/ionization (SELDI), high performance liquid chromatography, mass spectrometry, chemiluminescence, nephelometry/turbometry, lateral flow or pure or polarized fluorescence or electrophoresis.

It will be appreciated that the sample of cells from the patient may be cancer cells or may be normal (non-cancerous cells). For example, the latter may be useful for detecting the presence of germline mutations associated with low or absent functional activity of SMARCB1 (SNF5, INI1).

Alternatively, or in addition, the assessment of the patient further may comprise measuring the amount of *SMARCB1* mRNA, e.g. by quantitative PCR, Northern blot analysis, deep sequencing, SAGE, or array technologies.

Alternatively, or in addition, the assessment of the patient further may comprise determining the level of SMARCB1 activity (either directly or indirectly). Such activity may be assayed indirectly, for example by determining the genomic DNA or cDNA sequence, e.g. by fluorescence in situ hybridization, comparative genomic hybridization (CGH), array CGH, other array technologies, or sequencing techniques. The sequence information may then be used to identify chromosomal aberrations or DNA mutations that lead to a reduced or lost SMARCB1 activity.

Alternatively, or in addition, the assessment of the patient further may comprise determining epigenetic alterations (e.g. DNA methylation, histone modifications), that lead to low or absent SMARCB1 gene expression e.g. by DNA methylation analyses, chromatin immunoprecipitation-based techniques, mass spectrometry, chemical reactions (e.g. bisulfite treatment), or methylation profiling used for tumor classification.Alternatively, elF2alpha phosphorylation and/or PP1 activity can be used as indirect markers of SMARCB1 activity.

However, it would be apparent to a person skilled in the art that this list of techniques is not complete and these techniques are not the only suitable methods which may be used in the present invention for measuring the functional activity (e.g. expression) of SMARCB1 (SNF5, INI1).

Thus, the assessment may comprise performing a biopsy to extract a sample of cancer cells from the patient, which cells can then be tested (either directly or indirectly as a primary cell culture) to determine the functional activity/function (e.g. expression) of SMARCB1 therein. Alternatively, or in addition, normal tissue or cells from the patient may be used to determine the functional activity (e.g. expression) of SMARCB1 therein as germline mutations have been found in patients with familial or sporadic tumors (see for example Sevenet et al., 1999, Am J Human Genet 65, 1342-8; Eaton et al., 2011; Pediatr Blood Cancer 56, 7-15).

However, persons of skill in the art will appreciate that the functional activity (e.g. expression) of SMARCB1 may be determined indirectly.

Thus, the assessment of the patient may comprise diagnosing the type of cancer from which the patient is suffering (using conventional methods well known in the art for cancer diagnosis). This diagnosis can then be used to determine the functional activity (e.g. expression) of SMARCB1 in the cancer cells (either through the empirical knowledge of the physician or by consulting a database of gene expression and gene function in known cancer types (such as Gene expression omnibus, ArrayExpress, SAGEmap, RefExA, caArrayData Portal, GeneX, HuGElndex, TCGA databases, RCGDB, International Cancer Genome Consortium databases, Mitelman database of Chromosome Aberrations and Gene Fusions in Cancer, SKY/M-FISH&CGH database, COSMIC, TmaDB, YMD, dbEST, TMAD, GXA, SMD, Novartis Gene Expression Database, OncoMine and similar databases). Upon determining that the cancer from which the patient is suffering is associated with (cancer) cells in which the function activity (e.g. expression) of SMARCBH1 is low or absent, the patient may be administered an inhibitor of the DHODH enzyme as a therapeutic agent to treat the cancer.

By 'inhibitor of the DHODH enzyme" we mean an agent, such as a small chemical entity, polypeptide or the like, which is capable of inhibiting (at least, in part) a function of the DHODH enzyme (preferably in vivo in humans). Such an inhibitor may act at any point along the DHODH pathway, for example by inhibiting (at least, in part) the enzymes of the *de novo* nucleotide synthesis pathways.

In one embodiment, the inhibitor of the DHODH enzyme is a DHODH inhibitor selected from the group consisting of BAY2402234, AG636/AUR-108, Farudodstat, GTX-916, GTX-196, ASLAN003, JNJ- 74856665, and PTC299.

It will be further appreciated by persons skilled in the art that the inhibitor of the DHODH enzyme may be formulated at various concentrations, depending on a number of factors including the efficacy/toxicity of the inhibitor being used and the indication for which it is being used. Of course, the maximum concentration in any given pharmaceutical formulation will be limited by the maximum solubility of the inhibitor therein. However, the formulations should contain an amount of the inhibitor sufficient to provide an in vivo concentration at or near the target cancer cells which is sufficient to induce their cell death (e.g. via apoptosis).

In one embodiment, the inhibitor of the DHODH inhibitor is formulated at a concentration of between 1 nM and 1 M. For example, the pharmaceutical formulation may comprise a DHODH inhibitor at a concentration of between 1 uM and 1 mM, for example between 1 uM and 100 pM, between 5 uM and 50 uM, between 10 uM and 50 pM, between 20 uM and 40 uM or about 30 uM.

The inhibitors of the DHODH enzyme will generally be administered in admixture with a suitable pharmaceutical excipient, diluent or carrier selected with regard to the intended route of administration and standard pharmaceutical practice (for example, see Remington: The Science and Practice of Pharmacy, 19" edition, 1995, Ed. Alfonso Gennaro, Mack Publishing Company, Pennsylvania, USA; incorporated herein by reference). Suitable routes of administration are discussed below, and include intravenous, oral, pulmonary, intranasal, topical, aural, ocular, bladder and CNS delivery such as, but not limited to, intrathecal and/or intraventricular delivery, for example via an Ommaya reservoir.

For example, the inhibitor of the DHODH enzyme may be administered orally, buccally or sublingually in the form of tablets, capsules, ovules, elixirs, solutions or suspensions, which may contain flavouring or colouring agents, for immediate-, delayed- or controlled-release applications. Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxy-propyicellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The formulations may alternatively be administered parenterally, for example, intravenously, intraarterially, intratumorally, peritumorally, intraperitoneally, intrathecally, intraventricularly, intrasternally, intracranially, intra-muscularly or subcutaneously (including via an array of fine needles or using needle-free Powderject^{®} technology), or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or muiti-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

The inhibitors of the DHODH enzyme may also be administered intranasally or by inhalation and are conveniently delivered in the form of a dry powder inhaler or an aerosol spray presentation from a pressurized container, pump, spray or nebulizer with the use of a suitable propellant, e.g. dichlorodifluoromethane, trichlorofluoromethane, dichlorotetra-fluoroethane, a hydrofluoroalkane such as 1,1,1,2-tetrafluoroethane (HFA 134A° or 1,1,1,2,3,3,3-heptaflucropropane (HFA227EA^{®}), carbon dioxide or other suitable gas. In the case of a pressurized aerosol, the dosage unit may be determined by providing a valve to deliver a metered amount. The pressurized container, pump, spray or nebulizer may contain a solution or suspension of the active DHODH inhibitor, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or "puff' contains at least 1 mg of a compound for delivery to the patient. It will be appreciated that the overall dose with an aerosol will vary from patient to patient and from indication to indication, and may be administered in a single dose or, more usually, in divided doses throughout the day.

Alternatively, other conventional administration routes known in the art may also be employed; for example the formulation of the invention may be delivered orally, buccally or sublingually in the form of tablets, capsules, ovules, elixirs, solutions or suspensions, which may contain flavoring or coloring agents, for immediate-, delayed- or controlled- release applications. The formulation may also be administered intra-ocularly, intra- aurally or via intracavernosal injection (see below).

For application topically, e.g. to the skin, the inhibitor of the DHODH enzyme can be administered in the form of a lotion, solution, cream, gel, ointment or dusting powder (for example, see Remington, supra, pages 1586 to 1597). Thus, the DHODH inhibitors can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, e-lauryl sulphate, an alcohol (e.g. ethanol, cetearyl alcohol, 2-octyldodecanol, benzyl alcohol) and water.

Formulations suitable for topical administration in the mouth further include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

The formulation may also be administered by the ocular route, particularly for treating diseases of the eye. For ophthalmic use, the compounds can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For veterinary use, a compound is administered as a suitably acceptable formulation in accordance with normal veterinary practice and the veterinary surgeon will determine the dosing regimen and route of administration which will be most appropriate for a particular animal.

In one particular embodiment, the formulation is suitable for systemic administration to a patient (for example, via an oral or parenteral administration route).

The formulation comprising the inhibitor of the DHODH enzyme may be stored in any suitable container or vessel known in the art. It will be appreciated by persons skilled in the art that the container or vessel should preferably be airtight and/or sterilized. Advantageously, the container or vessel is made of a plastics material, such as polyethylene.

In one embodiment, the inhibitor of DHODH is for administration at a dose sufficient to induce cell death (e.g. apoptosis) of cancer cells in the patient being treated. Thus, the dose of the DHODH inhibitor may be chosen in order to inhibit the growth and/or number of cancer cells in the patient.

It will be appreciated that the dose of inhibitor of the DHODH enzyme may be changed during the course of treatment of the patient. For example, a higher dose may be used during an initial therapeutic treatment phase of an existing cancer, followed by a lower 'maintenance' dose after the initial treatment is complete to prevent recurrence of the cancer.

In one embodiment, the inhibitor of the DHODH enzyme is for administration at a dose of between 4.0 to 500 mg/kg per dose. For example, the DHODH inhibitor may be administered at a dose of between 4 mg/kg and 250 mg/kg, for example between 4 mg/kg and 100 mg/kg, between 4 mg/kg and 50 mg/kg, between 4 mg/kg and 25 mg/kg, between 4 mg/kg and 10 mg/kg or about 8 mg/kg, which may be repeated at regular intervals (for example daily, twice weekly, weekly, bi-weekly, monthly, etc).

It will be appreciated by persons skilled in the art that the inhibitor of the DHODH enzyme may be for use as a sole treatment for cancer in a patient or as part of a combination treatment (which further treatment may be a pharmaceutical agent, radiotherapy and/or surgery). Thus, the patient may also receive one or more further treatments for cancer, for example pharmaceutical agents (such as chemotherapeutic agents), radiotherapy and/or surgery.

### Definitions

Various terms relating to the methods, compositions, formulations, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

Methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

"A," "an," and "the": these singular form terms include plural referents unless the content clearly dictates otherwise. The indefinite article "a" or "an" thus usually means "at least one". Thus, for example, reference to "a cell" includes a combination of two or more cells, and the like.

"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods. Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth.

"And/or": The term "and/or" refers to a situation wherein one or more of the stated cases may occur, alone or in combination with at least one of the stated cases, up to with all of the stated cases.

"Comprising": this term is construed as being inclusive and open ended, and not exclusive. Specifically, the term and variations thereof mean the specified features, steps or components are included. These terms are not to be interpreted to exclude the presence of other features, steps or components.

Exemplary": this terms means "serving as an example, instance, or illustration," and should not be construed as excluding other configurations disclosed herein.

The term "expression" may refer to the transcription and/or translation of a particular nucleotide sequence driven by a promoter.

The terms "protein" and "polypeptide" are used interchangeably and may refer to any polymer of amino acids (dipeptide or greater) linked through peptide bonds or modified peptide bonds. Polypeptides of less than about 10-20 amino acid residues are commonly referred to as "peptides." The polypeptides of the invention may comprise non-peptidic components, such as carbohydrate groups. Carbohydrates and other non-peptidic substituents may be added to a polypeptide by the cell in which the polypeptide is produced, and will vary with the type of cell. Polypeptides are defined herein, in terms of their amino acid backbone structures; substituents such as carbohydrate groups are generally not specified, but may be present nonetheless.

The terms "reduced" or "eliminated" or "disruption" or "disrupted" are used interchangeably herein to refer to any genetic modification that decreases or eliminates expression and/or the functional activity of the nucleic acid or an expression product thereof. For example, disruption of a gene includes within its scope any genetic modification that decreases or eliminates expression of the gene and/or the functional activity of a corresponding gene product ( e.g ., mRNA and/or protein). Genetic modifications include complete or partial inactivation, suppression, deletion, interruption, blockage, or down- regulation of a nucleic acid (e.g., a gene). Illustrative genetic modifications include, but are not limited to, gene knockout, inactivation, mutation (e.g., insertion, deletion, point, or frameshift mutations that disrupt the expression or activity of the gene product), or use of inhibitory nucleic acids (e.g., inhibitory RNAs such as sense or antisense RNAs, molecules that mediate RNA interference such as siRNA, shRNA, miRNA; etc.), inhibitory polypeptides (e.g., antibodies, polypeptide-binding partners, dominant negative polypeptides, enzymes etc.) or any other molecule that inhibits the activity of the LCK gene or level or functional activity of an expression product of the LCK gene.

By "pharmaceutically acceptable carrier" is meant a solid or liquid filler, diluent or encapsulating substance that can be safely used in topical or systemic administration to an animal, preferably a mammal, including humans. Representative pharmaceutically acceptable carriers include any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329, incorporated herein by reference). Except insofar as any conventional carrier is incompatible with the active ingredient(s), its use in the pharmaceutical compositions is contemplated.

The term "cancer" is defined as, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation. In some embodiments, the tumor antigen expressing cells express, or at any time expressed, mRNA encoding the tumor antigen. In an embodiment, the tumor antigen-expressing cells produce the tumor antigen protein (e.g., wild- type or mutant), and the tumor antigen protein may be present at normal levels or reduced levels. In an embodiment, the tumor antigen-expressing cells produced detectable levels of a tumor antigen protein at one point, and subsequently produced substantially no detectable tumor antigen protein. In an embodiment, the tumor antigen-expressing cells overexpresses the tumor antigen protein.

The term Rhabdoid tumor (RT) refers to an aggressive (pediatric) soft tissue sarcoma that arises in the kidney, the liver, the peripheral nerves and all miscellaneous soft parts throughout the body. RT involving the central nervous system (CNS) is called atypical teratoid rhabdoid tumor. Atypical teratoid/rhabdoid tumor (AT/RT) of the CNS is an extremely rare and aggressive tumor of - typically - early childhood. The poor outcome with conventional infant brain tumor therapy has resulted in a lack of clear treatment guidelines.

Central nervous system AT/RTs typically demonstrate a variety of primitive neuroectodermal, epithelial or mesenchymal cells, which underlies the difficulty in distinguishing these tumors from other primitive neuroectodermal tumors or choroid plexus carcinomas. Immunohistochemistry is often used in the differential diagnosis, based on the typical expression of smooth muscle actin, epithelial membrane antigen and vimentin.

The vast majority of rhabdoid tumors contain bi-allelic inactivating mutations in the SMARCB1 gene. Lack of expression of the SMARCB1 protein is also employed as a specific means of distinguishing rhabdoid tumors from other malignancies with similar histologic features, especially for diagnosis of AT/RT versus primitive neuroectodermal tumors. Individuals with germline alterations of SMARCB1 are predisposed to rhabdoid tumors of the brain, kidney and soft tissues and may present with more than one primary tumor. These children are most often diagnosed within the first year of life and tend to have a worse prognosis. It is not known whether the poor prognosis is related to the presence of a germline mutation in ail of their cells, or the fact that they develop multiple and progressive primary tumors that are resistant to therapy.

SMARCB1 (SWI/SNF-related matrix-associated actin-dependent regulator of chromatin subfamily B member 1), a.k.a. INI1 (Integrase Interactor 1), is a subunit of the mammalian SWI/SNF (SWItch/Sucrose Non-Fermentable) ATP-dependent chromatin remodeling complexes. SWI/SNF complexes, also known as BRG1/BRM-associated factor (BAF) complexes, are central regulators of nucleosome remodeling.

Mammalian SWI/SNF complexes are classified into three subgroups: canonical BAF (cBAF), polybromo-associated BAF (PBAF), and non-canonical BAF (ncBAF), also called GLTSCR1 or GLTSCR1L-containing and BRD9-containing (GBAF) complexes. Some subunits and the ATPases are shared between all three subfamilies (e.g., SMARCC1, SMARCC2, SMARCD1, SMARCA4, SMARCA2), whereas other components are specific for each subgroup. SMARCB1 participates only into cBAF and PBAF complexes. SWI/SNF complexes are involved in numerous biological processes, including cell cycle regulation and maintenance of genomic stability, and it has been estimated that alterations in SWI/SNF subunits involve over 20% of all cancers.

. The protein SMARCB1 is highly conserved, as evidenced by an identical amino acid sequence in mice and humans. However, the function of SMARCB 1 is poorly understood. There are no SMARCB 1 paralogs and the protein lacks particularly informative protein motifs.

Accordingly, there is a need for therapies for the treatment of cancers such as rhabdoid tumors, as well as method for selecting an effective treatment regime in cancer patients.

The term "administering" refers to contacting, applying, injecting, transfusing or providing a composition of the present invention to a subject.

The term "treating" as used herein may refer to (1) preventing or delaying the appearance of one or more symptoms of the disorder; (2) inhibiting the development of the disorder or one or more symptoms of the disorder; (3) relieving the disorder, i.e., causing regression of the disorder or at least one or more symptoms of the disorder; and/or (4) causing a decrease in the severity of one or more symptoms of the disorder.

The term "subject" or "patient" as used throughout the specification is to be understood to mean a human or may be a domestic or companion animal. While it is particularly contemplated that the methods of the invention are for treatment of humans, they are also applicable to veterinary treatments, including treatment of companion animals such as dogs and cats, and domestic animals such as horses, cattle and sheep, or zoo animals such as primates, felids, canids, bovids, and ungulates. The "subject" may include a person, a patient or individual, and may be of any age or gender.

The term "a therapeutically effective amount" of a compound (e.g. chemical entity or biologic agent) of the present disclosure refers to an amount of the compound of the present disclosure that will elicit the biological or medical response of a subject, for example, reduction or inhibition of an enzyme or a protein activity, or ameliorate symptoms, alleviate conditions, slow or delay disease progression, or prevent a disease, etc. In one embodiment a therapeutically effective amount in vivo may range depending on the route of administration, between about 0.1-500 mg/kg, or between about 1-100 mg/kg.

The optimal dosage of each combination partner for treatment of a cancer can be determined empirically for each individual using known methods and will depend upon a variety of factors, including, though not limited to, the degree of advancement of the disease; the age, body weight, general health, gender and diet of the individual; the time and route of administration; and other medications the individual is taking. Optimal dosages may be established using routine testing and procedures that are well known in the art. The amount of each combination partner that may be combined with the carrier materials to produce a single dosage form will vary depending upon the individual treated and the particular mode of administration. In some embodiments the unit dosage forms containing the combination of agents as described herein will contain the amounts of each agent of the combination that are typically administered when the agents are administered alone. Frequency of dosage may vary depending on the compound used and the particular condition to be treated or prevented. In general, the use of the minimum dosage that is sufficient to provide effective therapy is preferred. Patients may generally be monitored for therapeutic effectiveness using assays suitable for the condition being treated or prevented, which will be familiar to those of ordinary skill in the art.

An effective dose of the DHODH inhibitor can be based in preclinical studies in mice (PROUS integrity records). The dose escalation study in man will allow to identify the maximum tolerated dose, and will allow to define the recommended clinical dose for pivotal clinical studies.

An effective dose of the DHODH inhibitor may range from about 4 mg to about 500 mg daily. For example, the DHODH inhibitor may be administered at a dose of between 4 mg/kg and 250 mg/kg, for example between 4 mg/kg and 100 mg/kg, between 4 mg/kg and 50 mg/kg, between 4 mg/kg and 25 mg/kg, between 4 mg/kg and 10 mg/kg or about 8 mg/kg.

### Detailed description

Brain cancer is one of the leading causes of cancer-associated deaths among children [1]. Current treatment modalities (eg, surgical resection, intensive chemotherapy, and radiation therapy) have shown limited effectiveness. Moreover, current treatment options often cause nonspecific cytotoxic effects that negatively affect the development of the young patient's brain, resulting in long-term neurological deficits. In particular, atypical teratoid rhabdoid tumor (AT/RT) is a rare but highly malignant form of brain cancer that predominantly affects children under three years [2,3]. The median age at diagnosis is approximately 17 months, and patients typically survive less than one year after diagnosis [2,4]. Currently, there is no definitive standard of care for AT/RT, and traditional treatments are not effective enough to stop aggressive progression of the disease. Given the dismal prognosis associated with ATRT, better treatment approaches are urgently needed to improve the treatment outcomes in these very young patients.

**Despite being part of many pediatric cancer treatment schedules, the use of MTX is associated with severe toxicity and a wide range of side effects.** Patients treated with MTX always receive close monitoring of their health and if required also leucovorin rescue therapy. As expected, daily dosing of MTX in ECRT-bearing mice in vivo is associated with toxicity, indicated by significant weight loss over time in some of the mice. Additionally, it has been shown that patients treated with MTX develop resistance for which the biological mechanisms contributing to the generation of this resistance in AT/RT are unknown. Therefore, there is a great medical need to find a treatment of rhabdoid tumors, including MRTs and AT/RTs, without causing this severe toxicity and wide range of side effects in patients.

As part of the invention herein, we identified targetable metabolic vulnerabilities in MRTs, extra-(ECRTs) and intra-cranial (AT/RTs) malignant rhabdoid tumors. We also demonstrated that, unexpectedly, targeting rhabdoid tumors with DHODH inhibitors show in vivo and in vitro efficacy in inhibiting tumor growth with much less toxicity and side effects than MTX.

The *DHODH* gene, located in the open reading frame (ORF) of human chromosome 16q22 with full length of 1191 bp, encodes DHODH protein with 397 amino acid sequences [5].

According to sequence similarity and subcellular location, DHODH is divided into Class 1 and Class 2 DHODHs. Soluble class 1 DHODHs are further classified into class 1A, class 1B, and class 1S, which are all located in cytoplasm. Class 1A are homodimeric proteins and found in Gram-positive bacteria. Class 1B DHODHs is a dimer of heterodimers and usually found in prevalent in Gram-positive bacteria, consisting of two distinct proteins. The S DHODH is a newly found type which is incapable of utilizing any of the natural electron acceptors. It uses serine as catalytic base, which is special for a cytosolic DHODH [6, 7]. Class 2 DHODHs are monomeric proteins that attach to the mitochondrial inner membrane in eukaryotes and some prokaryotes [7-10]. Class 1A and class 1B share approximately 30% sequence identity, whereas soluble class 1 and membrane-bound class 2 DHODHs share approximately 20% of sequence identity [7].

Pyrimidines are necessary for the biosynthesis of DNA, RNA, glycoproteins and phospholipids [11]. Pyrimidine nucleotides are synthesized through two pathways: the de novo synthesis pathway and the salvage pathway [12]. Pyrimidines are synthesized de novo from simple precursors with six steps. The enzymes that catalyze uridine monophosphate (UMP) synthesis include carbamoyl phosphate synthetase II (CPSII), aspartate transcarbamoylase (ATCase), dihydroorotase (DHOase), DHODH, and uridine monophosphate synthase (UMPS) [13]. Firstly, glutamine (Gln), ATP and HCO₃- collectively form carbamoyl phosphate, which is catalyzed by CPSII, a vital enzyme located in the cytosol. ATCase contributes to the formation of the carbamoyl-aspartate following, the pyrimidine ring further is cyclized by DHOase to produce dihydroorotate. Secondly, the flavoenzyme DHODH converts dihydroorotate to orotate which takes place in mitochondria. Orotate further converts to UMP by the UMP synthase UMPS. UMP, a precursor of other pyrimidines and various biological processes, then converts to uridine triphosphate (UTP) by phosphorylation. UTP is subsequently converted to cytidine triphosphate (CTP) with the donor glutamine by CTP synthetase. Meanwhile, the uridine diphosphate (UDP) transforms to deoxyuridine diphosphate (dUDP) by ribonucleotide reductase, with its ribose moiety reducing to deoxyribose. Deoxyuridine monophosphate (dUMP) methylation generates deoxythymine nucleotides (dTMP or TMP) for DNA synthesis [14, 15-17]. In this process, DHODH catalyzes the fourth step in the de novo biosynthesis of pyrimidine by converting dihydroorotate into orotate in a redox reaction in the mitochondria with ubiquinone (CoQ) converting to ubiquinol (CoQH2), which is a substrate of respiratory complex III [14, 7, 18].

As DHODH plays a crucial part in pyrimidine synthesis, it exerts varying effects on different cell types and developmental stages owing to the relative contribution of de novo pyrimidine synthesis to maintaining proliferation. The de novo pyrimidine synthesis has gained a prominent position to satisfy increasing demand for nucleic acid precursors in rapidly proliferative cells such as activated T cells due to the rapidly increased demand for DNA replication and nucleic acid biosynthesis [11, 15, 19, 20]. While in resting or fully differentiated cells, they mainly obtain pyrimidines through the salvage pathway for proliferation [11, 21-24]. Thus, the effect of DHODH is more predominate in rapidly proliferating cells like cancer cells, which may be highly sensitive to inhibition of nucleotide synthesis [25-27].

Consistent with above observations, DHODH blockade by inhibitors or RNA interference exhibited anti-proliferation effect by pyrimidine depletion. Studies manifest that DHODH inhibitors have more potential to treat malignancies which are more dependent on de novo pyrimidine synthesis and have lower pyrimidine salvage activity. For instance, in PTEN-mutant cells which depend upon glutamine flux through the de novo pyrimidine synthesis pathway [28], inhibition of DHODH causes stalled forks due to inadequate nucleotide pools required to support replication. Sustained treatment with DHODH inhibitor further leads to Rad3-related kinase (ATR) activation, leading to a buildup of DNA damage and cell death [28]. Cancer cells are hypersensitive to DHODH inhibitors under the tumor hypoxia and nutrient-deprived microenvironment [19, 29]. Further, depletion of the pyrimidine nucleotide pool, especially UTP, resulting from DHODH inhibition, could impair biogenesis of ribosomes, which activates the tumor suppressor p53 pathway leading to cell cycle arrest [30, 31]. Moreover, DHODH is regulated by several critical transcription factors [16]. The predicted and known regulators of DHODH include E1A-binding protein p300, POU domain, class 3, transcription factor 2 (POU3F2), GATA-binding factor 2 (GATA-2), nuclear factor kappa-light-chain-enhancer of activated B cells 1 (NF-κB1), and proto-oncogene MYC [16]. It might be significant to inhibit DHODH through interfere with these regulatory factors, which can further contribute to pyrimidine depletion and induce death of tumor cells.

Apart from the direct blockade of pyrimidine synthesis, DHODH also exerts additional effects including O-linked N-acetylglucosaminylation (O-GlcNAc) [32], senescence [33], AND mRNA translation [34] indirectly by pyrimidine depletion. UMP, the downstream product of DHODH, is a precursor for components required for the assembly of various cellular macromolecules, including phospholipids, glycogen, hyaluronic acid, and proteoglycans, as well as for certain post-translational protein modifications [23, 35], which may account for the above extensive influences of DHODH. For example, O-GlcNAc, a post-translational modification of proteins, can add *N-*acetylglucosamine (GicNAc) on UDP [36]. UMP reduction by DHODH suppression may result in integral decreases in protein *N*-acetyl glycosylation in acute myeloid leukemia (AML) [37], and reduction of UMP-GlcNAc promotes myeloid differentiation [37]. Given that O-GlcNAc plays a crucial role in myeloid differentiation [38-40], it may explain the mechanism that DHODH inhibition promotes the myeloid differentiation in AML to some extent. Collectively, DHODH inhibition decreases the de novo pyrimidine synthesis and affects many biological processes.

Despite robust preclinical anticancer efficacy, DHODH inhibitors have shown limited single-agent activity in phase 1 and 2 clinical trials. In the invention herein, we demonstrate that even though, based on the prior art, the use of DHODH inhibitors would not be an obvious choice for the treatment of cancer, our results in vitro in patient-derived organoid (PDO) and in vivo demonstrated a metabolic dependency to DHODH inhibition in rhabdoid tumors, which was not expected, targetable metabolic vulnerabilities in extra- (ECRTs) and intra-cranial (AT/RTs) malignant rhabdoid tumors. Targeting of de novo pyrimidine synthesis via DHODH inhibitors show in vivo/ in vitro efficacy in inhibiting/controlling tumor growth. In xenografts methotrexate (MTX) has been tested. MTX- and BAY-2402234 induced cytotoxicity seems to be tumor-specific in vitro.

Although genomic instability is a common feature of most malignant cells [41], AT/RT genomes are highly stable. Most AT/RTs contain less than ten coding mutations, and large chromosome gain or loss is uncommon [42-44]. The only recurring molecular abnormality that characterizes AT/RT is the inactivation of the SMARCB1 gene, which is observed in nearly 98% of AT/RT patients [45]. SMARCB1 encodes a core subunit of the SWI/SNF chromatin remodeling complex that contributes to developmental processes. Although up to 20% of human cancers contain a loss-of-function mutation in the SWI/SNF complex, its role in tumor suppression remains understudied [46]. It was only recent that several studies have revealed that SMARCB1 is a tumor suppressor with its loss required for rhabdoid tumorigenesis [3,46]. The precise mechanism through which SMARCB1 can act as a tumor suppressor remains to be fully understood [43,46-50]. In rhabdoid tumors, inactivation of SMARCB1 can drive proliferation by altering the expression of multiple pro-oncogenic pathways [51-57]. Importantly, loss of SMARCB1 also reduces the efficacy of conventional anti-cancer treatments, such as radiation and chemotherapy [58,59]. Thus, restoration of SMARCB1 is an attractive approach for treating patients with ATRT with the potential to render their ATRT more sensitive to conventional therapeutic modalities.

Combining emerging knowledge regarding the role of SMARCB1 in a broad range rhabdoid tumors, using the inventors' expertise and particular models of MRT and AT/RT as well as their knowledge in the role of DHODH inhibition in MRT and AT/RT tumors, the present invention relates to a novel use of DHODH inhibitors for the treatment of cancer in a patient. In particular, there are provided agents and methods for use in the treatment of cancer wherein the level of functional activity of SMARCB1 in the cancer cells is used to determine whether the patient would benefit from treatment with an inhibitor of the DHODH enzyme.

### References

1. Siegel RL, Miller KD, JemalA. Cancer statistics, 2018. CA Cancer J Clin. 2018;68(1):7-30.
2. Lau CS, Mahendraraj K, Chamberlain RS. Atypical teratoid rhabdoid tumors: a population-based clinical outcomes study involving 174 patients from the Surveillance, Epidemiology, and End Results database (1973-2010). Cancer Manag Res. 2015;7:301-309.
3. Ginn KF, Gajjar A. Atypical teratoid rhabdoid tumor: current therapy and future directions. Front Oncol. 2012;2:114.
4. Lafay-Cousin L, Hawkins C, Carret AS, et al. Central nervous system atypical teratoid rhabdoid tumours: the Canadian Paediatric Brain Tumour Consortium experience. Eur J Cancer. 2012;48(3):353-359.
5. Barnes T, Parry P, Hart I, Jones C, Minet M, Patterson D. Regional mapping of the gene encoding dihydroorotate dehydrogenase, an enzyme involved in UMP synthesis, electron transport, and superoxide generation, to human chromosome region 16q22. Somat Cell Mol Genet. 1993;19:405-411. doi: 10.1007/BF01232751.
6. Sorensen PG, Dandanell G. A new type of dihydroorotate dehydrogenase, type 1S, from the thermoacidophilic archaeon Sulfolobus solfataricus. Extremophiles. 2002;6(3):245-251. doi: 10.1007/s00792-001-0249-0.
7. Reis RAG, Calil FA, Feliciano PR, Pinheiro MP, Nonato MC. The dihydroorotate dehydrogenases: past and present. Arch Biochem Biophys. 2017;632:175-191. doi: 10.1016/j.abb.2017.06.019.
8. Björnberg O, Grüner AC, Roepstorff P, Jensen KF. The activity of Escherichia coli dihydroorotate dehydrogenase is dependent on a conserved loop identified by sequence homology, mutagenesis, and limited proteolysis. Biochemistry. 1999;38:2899-2908. doi: 10.1021/bi982352c.
9. Palfey BA, Björnberg O, Jensen KF. Insight into the chemistry of flavin reduction and oxidation in Escherichia coli dihydroorotate dehydrogenase obtained by rapid reaction studies. Biochemistry. 2001;40:4381-4390. doi: 10.1021/bi0025666.
10. Hey-Mogensen M, Goncalves RLS, Orr AL, Brand MD. Production of superoxide/H2O2 by dihydroorotate dehydrogenase in rat skeletal muscle mitochondria. Free Radic Biol Med. 2014;72:149-155. doi: 10.1016/j.freeradbiomed.2014.04.007.
11. Vyas V, Ghate M. Recent developments in the medicinal chemistry and therapeutic potential of dihydroorotate dehydrogenase (DHODH) Inhibitors. Mini-Rev Med Chem. 2011;11:1039-1055. doi: 10.2174/138955711797247707.
12. Fang J, Uchiumi T, Yagi M, Matsumoto S, Amamoto R, Takazaki S, et al. Dihydro-orotate dehydrogenase is physically associated with the respiratory complex and its loss leads to mitochondrial dysfunction. Biosci Rep. 2013;33:217-227.
13. Löffler M, Fairbanks LD, Zameitat E, Marinaki AM, Simmonds HA. Pyrimidine pathways in health and disease. Trends Mol Med. 2005;11:430-437. doi: 10.1016/j.molmed.2005.07.003.
14. Evans DR, Guy HI. Mammalian pyrimidine biosynthesis: fresh insights into an ancient pathway. J Biol Chem. 2004;279:33035-33038. doi: 10.1074/jbc.R400007200.
15. Löffler M, Fairbanks LD, Zameitat E, Marinaki AM, Simmonds HA. Pyrimidine pathways in health and disease. Trends Mol Med. 2005;11:430-437. doi: 10.1016/j.molmed.2005.07.003.
16. Lane AN, Fan TWM. Regulation of mammalian nucleotide metabolism and biosynthesis. NucleicAcids Res. 2015;43:2466-2485. doi: 10.1093/nar/gkv047.
17. Wang X, Yang K, Wu Q, Kim LJY, Morton AR, Gimple RC, et al. Targeting pyrimidine synthesis accentuates molecular therapy response in glioblastoma stem cells. Sci Transl Med. 2019;11:1-15. doi: 10.1126/scitranslmed.aau4972.
18. Löffler M, Jöckel J, Schuster G, Becker C. Dihydroorotat-ubiquinone oxidoreductase links mitochondria in the biosynthesis of pyrimidine nucleotides. Mol Cell Biochem. 1997;174:125-129. doi: 10.1023/A:1006859115450.
19. Brown JM, Giaccia AJ. The unique physiology of solid tumors: opportunities (and problems) for cancer therapy. Cancer Res. 1998;58:1408-1416.
20. Bofill M. Importance of Ribonucleotide Availability to Proliferating Tlymphocytes from Healthy Humans. J Biol Chem. 1995;270:29682-29689. doi: 10.1074/jbc.270.50.29690.
21. 28. Bofill M. Importance of Ribonucleotide Availability to Proliferating Tlymphocytes from Healthy Humans. J Biol Chem. 1995;270:29682-29689. doi: 10.1074/jbc.270.50.29690.
22. Mathur D, Stratikopoulos E, Ozturk S, Steinbach N, Pegno S, Schoenfeld S, et al. PTEN regulates glutamine flux to pyrimidine synthesis and sensitivity to dihydroorotate dehydrogenase inhibition. Cancer Discov. 2017;7:380-390. doi: 10.1158/2159-8290.CD-16-0612
23. Lewis TA, Sykes DB, Law JM, Muñoz B, Rustiguel JK, Nonato MC, Scadden DT, Schreiber SL. Development of ML390: a human DHODH inhibitor that induces differentiation in acute myeloid leukemia. ACS Med Chem Lett. 2016;7(12):1112-1117. doi: 10.1021/acsmedchemlett.6b00316.
24. Mascia L, Turchi G, Bemi V, Ipata PL. Uracil salvage pathway in PC12 cells. Biochim Biophys Acta, Gen Subj. 2000;1524:45-50. doi: 10.1016/S0304-4165(00)00139-2.
25. Huisman WH, Raivio KO, Becker MA. Simultaneous determination of rates of purine and pyrimidine synthesis in cultured human lymphoblasts and fibroblasts. Adv Exp Med Biol. 1979;122(B):223-229.
26. Jackson RC, Lui MS, Boritzki TJ, Weber G, Morris HP. Purine and pyrimidine nucleotide patterns of normal, differentiating, and regenerating liver and of hepatomas in rats. Cancer Res. 1980;40:1286-1291.
27. Sigoillot FD, Berkowski JA, Sigoillot SM, Kotsis DH, Guy HI. Cell cycle-dependent regulation of pyrimidine biosynthesis. J Biol Chem. 2003;278:3403-3409. doi: 10.1074/jbc.M211078200.
28. Mathur D, Stratikopoulos E, Ozturk S, Steinbach N, Pegno S, Schoenfeld S, et al. PTEN regulates glutamine flux to pyrimidine synthesis and sensitivity to dihydroorotate dehydrogenase inhibition. Cancer Discov. 2017;7:380-390. doi: 10.1158/2159-8290.CD-16-0612.
29. Miyazaki Y, Inaoka DK, Shiba T, Saimoto H, Sakura T, Amalia E, et al. Selective cytotoxicity of dihydroorotate dehydrogenase inhibitors to human cancer cells under hypoxia and nutrient-deprived conditions. Front Pharmacol. 2018;9:1-13. doi: 10.3389/fphar.2018.00997.
30. Hubackova S, Davidova E, Boukalova S, Kovarova J, Bajzikova M, Coelho A, et al. Replication and ribosomal stress induced by targeting pyrimidine synthesis and cellular checkpoints suppress p53-deficient tumors. Cell Death Dis. 2020;11.
31. Zhang Y, Lu H. Signaling to p53: ribosomal proteins find their way. Cancer Cell. 2009;16:369-377. doi: 10.1016/j.ccr.2009.09.024.
32. Sykes DB, Kfoury YS, Mercier FE, Wawer MJ, Law JM, Haynes MK, et al. Inhibition of dihydroorotate dehydrogenase overcomes differentiation blockade in acute myeloid leukemia. Cell. 2016;167:171-86.e15. doi: 10.1016/j.cell.2016.08.057.
33. Boukalova S, Hubackova S, Milosevic M, Ezrova Z, Neuzil J, Rohlena J. Dihydroorotate dehydrogenase in oxidative phosphorylation and cancer. Biochim Biophys Acta Mol basis Dis. 2020:165759.
34. Cao L, Weetall M, Trotta C, Cintron K, Ma J, Kim MJ, et al. Targeting of hematologic malignancies with PTC299, a novel potent inhibitor of dihydroorotate dehydrogenase with favorable pharmaceutical properties. Mol Cancer Ther. 2019;18:3-16. doi: 10.1158/1535-7163.MCT-18-0863.
35. Li L, Ng SR, Colón CI, Drapkin BJ, Hsu PP, Li Z, et al. Identification of DHODH as a therapeutic target in small cell lung cancer. Sci Transl Med. 2019;11:517.
36. Fardini Y, Dehennaut V, Lefebvre T, Issad T. O-GlcNAcylation: a new cancer hallmark? Front Endocrinol. 2013;4:1-15. doi: 10.3389/fendo.2013.00099.
37. Christian S, Merz C, Evans L, Gradl S, Seidel H, Friberg A, et al. The novel dihydroorotate dehydrogenase (DHODH) inhibitor BAY 2402234 triggers differentiation and is effective in the treatment of myeloid malignancies. Leukemia. 2019;33:2403-2415. doi: 10.1038/s41375-019-0461-5.
38. Ishihara K, Takahashi I, Tsuchiya Y, Hasegawa M, Kamemura K. Characteristic increase in nucleocytoplasmic protein glycosylation by O-GlcNAc in 3 T3-L1 adipocyte differentiation. Biochem Biophys Res Commun. 2010;398:489-494. doi: 10.1016/j.bbrc.2010.06.105.
39. Andrés-Bergós J, Tardio L, Larranaga-Vera A, Gómez R, Herrero-Beaumont G, Largo R. The increase in O-linked N-acetylglucosamine protein modification stimulates chondrogenic differentiation both in vitro and in vivo. J Biol Chem. 2012;287:33615-33628. doi: 10.1074/jbc.M112.354241.
40. Sun C, Shang J, Yao Y, Yin X, Liu M, Liu H, Zhou Y. O-GlcNAcylation: a bridge between glucose and cell differentiation. J Cell Mol Med. 2016;20(5):769-781. doi: 10.1111/jcmm.12807.
41. Hanahan D, Weinberg RA. Hallmarks of cancer: the next generation. Cell. 2011;144(5):646-674.
42. Douglass EC, Valentine M, Rowe ST, et al. Malignant rhabdoid tumor: a highly malignant childhood tumor with minimal karyotypic changes. Genes Chromosomes Cancer. 1990;2(3):210-216.
43. Hasselblatt M, Isken S, Linge A, et al. High-resolution genomic analysis suggests the absence of recurrent genomic alterations other than SMARCB1 aberrations in atypical teratoid/rhabdoid tumors. Genes Chromosomes Cancer. 2013;52(2):185-190.
44. Lee RS, Stewart C, Carter SL, et al. A remarkably simple genome underlies highly malignant pediatric rhabdoid cancers. J Clin Invest. 2012;122(8):2983-2988.
45. Alimova I, Pierce A, Danis E, et al. Inhibition of MYC attenuates tumor cell self-renewal and promotes senescence in SMARCB1-deficient Group 2 atypical teratoid rhabdoid tumors to suppress tumor growth in vivo. Int j Cancer. 2019;144(8):1983-1995.
46. Biegel JA, Tan L, Zhang F, Wainwright L, Russo P, Rorke LB. Alterations of the hSNF5/INI1 gene in central nervous system atypical teratoid/rhabdoid tumors and renal and extrarenal rhabdoid tumors. Clini Cancer Res. 2002;8(11):3461-3467.
47. Mathur R, Roberts CWM. SWI/SNF (BAF) Complexes: guardians of the Epigenome. Annu Rev Canc Biol. 2018;2:413-427.
48. Biegel JA, Allen CS, Kawasaki K, Shimizu N, Budarf ML, Bell CJ. Narrowing the critical region for a rhabdoid tumor locus in 22q11. Genes Chromosomes Cancer. 1996;16(2):94-105.
49. Torchia J, Golbourn B, Feng S, et al. Integrated (epi)-Genomic Analyses Identify Subgroup-Specific Therapeutic Targets in CNS Rhabdoid Tumors. Cancer Cell. 2016;30(6):891-908.
50. 15. Versteege I, Sevenet N, Lange J, et al. Truncating mutations of hSNF5/INI1 in aggressive paediatric cancer. Nature. 1998;394:6689.
51. Chasse MH, Johnson BK, Boguslawski EA, et al. Mithramycin induces promoter reprogramming and differentiation of rhabdoid tumor. EMBO Mol. Med. 2021;13(2).
52. Betz BL, Strobeck MW, Reisman DN, Knudsen ES, Weissman BE. Re-expression of hSNF5/INI1/BAF47 in pediatric tumor cells leads to G1 arrest associated with induction of p16ink4a and activation of RB. Oncogene. 2002;21(34):5193-5203.
53. Isakoff MS, Sansam CG, Tamayo P, et al. Inactivation of the Snf5 tumor suppressor stimulates cell cycle progression and cooperates with p53 loss in oncogenic transformation. Proc Natl Acad Sci USA. 2005;102(49):17745-17750.
54. Lee D, Kim JW, Seo T, Hwang SG, Choi EJ, Choe J. SWI/SNF complex interacts with tumor suppressor p53 and is necessary for the activation of p53-mediated transcription. J Biol Chem. 2002;277(25):22330-22337.
55. Lee S, Cimica V, Ramachandra N, Zagzag D, Kalpana GV. Aurora A is a repressed effector target of the chromatin remodeling protein INI1/hSNF5 required for rhabdoid tumor cell survival. Cancer Res. 2011;71(9):3225-3235.
56. Oruetxebarria I, Venturini F, Kekarainen T, et al. P16INK4a is required for hSNF5 chromatin remodeler-induced cellular senescence in malignant rhabdoid tumor cells. J Biol Chem. 2004;279(5):3807-3816.
57. Versteege I, Medjkane S, Rouillard D, Delattre O. A key role of the hSNF5/INI1 tumour suppressor in the control of the G1-S transition of the cell cycle. Oncogene. 2002;21(42):6403-6412.
58. Fruhwald MC, Biegel JA, Bourdeaut F, Roberts CW, Chi SN. Atypical teratoid/rhabdoid tumors-current concepts, advances in biology, and potential future therapies. Neuro-Oncology. 2016;18(6):764-778.
59. Lee YE, Choi SA, Kwack PA, et al. Repositioning disulfiram as a radiosensitizer against atypical teratoid/rhabdoid tumor. Neuro-Oncology. 2017;19(8):1079-1087.

### Figure legends

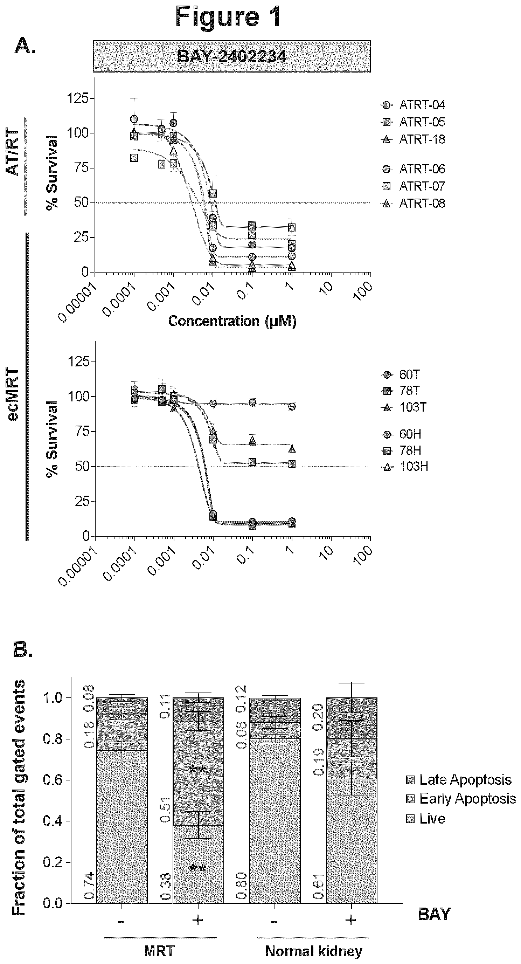

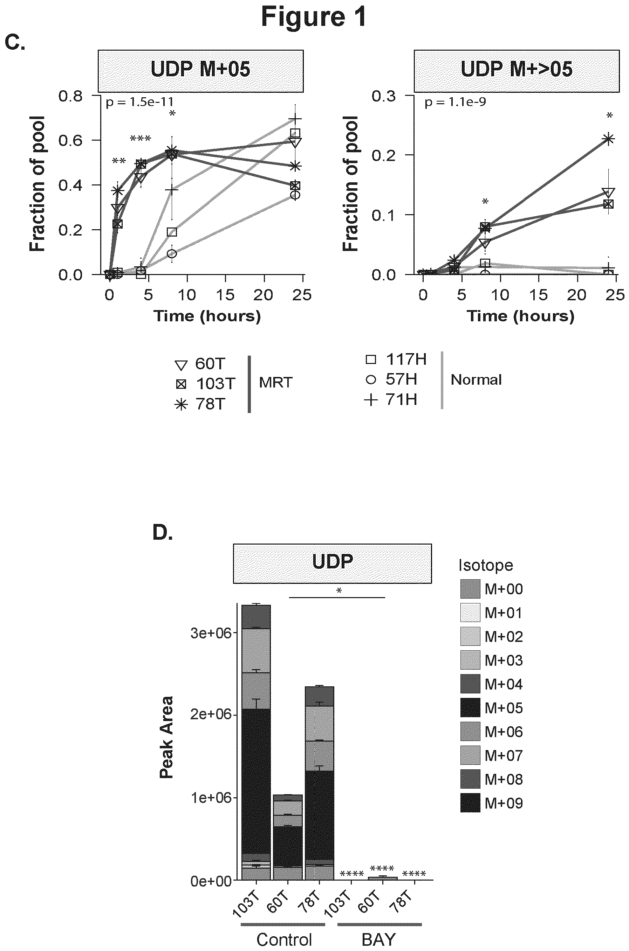

**Figure 1** - The de novo nucleotide synthesis is a metabolic vulnerability of rhabdoid tumors that can be targeted with DHODHi BAY-2402234. A) Dose-response curves of BAY-2402234 for the indicated organoid and tumoroid cultures. Data points are represented as the mean ± SD of three independent experiments, each consisting of quadruplicate measurements. Data are normalized to DMSO vehicle (100%). The grey dashed horizontal line represents a viability of 50% (IC50). B) Bar graphs representing live, early apoptotic, and late apoptotic cell fractions of MRT and normal kidney organoids upon treatment with 50 nM BAY for 120 h. The means ± SEM of n = 3 kidney organoid models are plotted. P values were generated by performing multiple paired Student's t test. C) Schematic overview of the incorporation of carbons from [U-13C6]-glucose into purines and pyrimidines. The ribose ring is produced via the pentose phosphate pathway (PPP; brown), resulting in UMP [M+5] and IMP [M+5]. The serine synthesis pathway (SSP) provides carbons for the purine nucleobase via glycine (light brown, [M+2]), or one-carbon metabolism (blue; [M+1]). Combinations of these pathways result in various purine labeling patterns. Aspartate (ASP) is providing carbons for the pyrimidine nucleobase (green; [M+3]), giving rise to the various pyrimidine labeling patterns. D) Incorporation of glucose-derived 13C in UDP over time in three normal kidney organoids and three MRT tumoroids after 24 hours of culturing in [U-13C6]-glucose. The effect of organoid type (normal kidney or MRT) on glucose incorporation over time was analyzed using a linear model with an interaction term of class and time. Significant interaction between class and time was defined as an improved model fit with interaction term of class and time over a model lacking this interaction. Student's t tests were performed on individual timepoints. E) Isotope distribution of UDP in three MRT tumoroids after 24 hours of culturing in [U-13C6]-glucose in the presence and absence of 5 nM BAY. Student's t test between Normal and MRT was performed on mean peak area of the sum of all isotopologues of individual cell lines. (*, p < 0.05; **, p < 0.01; ***, p< 0.001; ****, p < 0.0001).

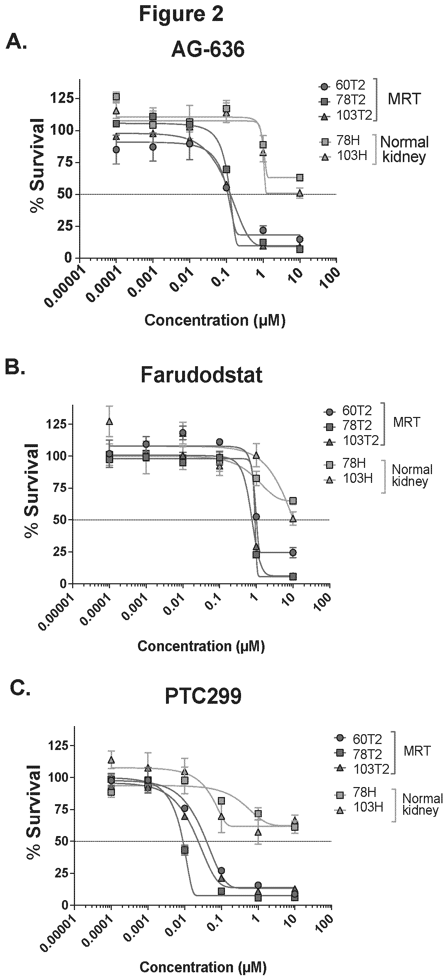

**Figure 2** - MRT tumoroids are sensitive to several DHODH inhibitors. A-C) Dose-response curves of DHODH inhibitors AG-636 (panel A), Farudodstat (panel B), and PTC299 (panel C) for the indicated normal kidney organoid and MRT tumoroid cultures. Data points are represented as the mean ± SD of quadruplicate measurements. Data are normalized to DMSO vehicle (100%). The grey dashed horizontal line represents a viability of 50% (IC50). D) Overview of the exact IC50 values of AG-636, Farudodstat, PTC299 and BAY-2402234 (as a reference) for each MRT tumoroid and normal kidney organoid model. IC50 values are given in µM.

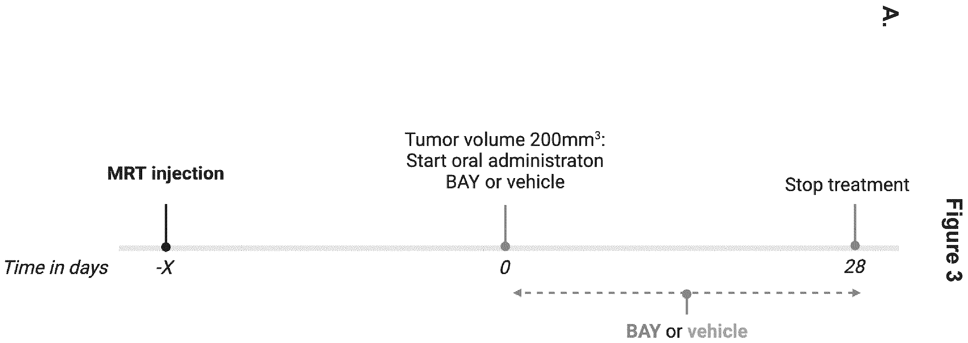

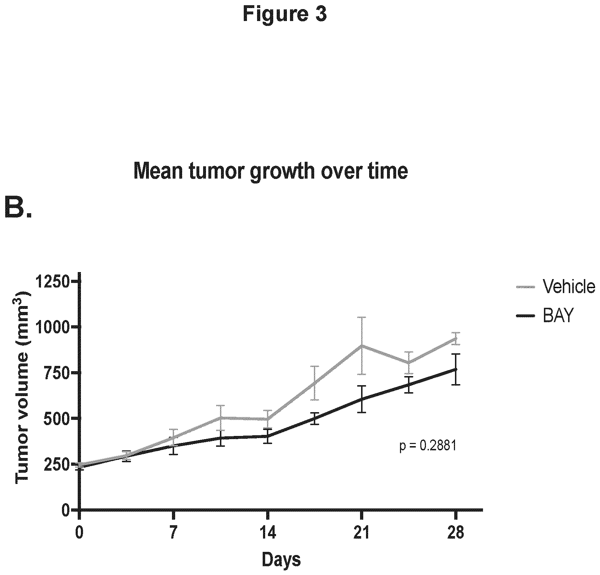

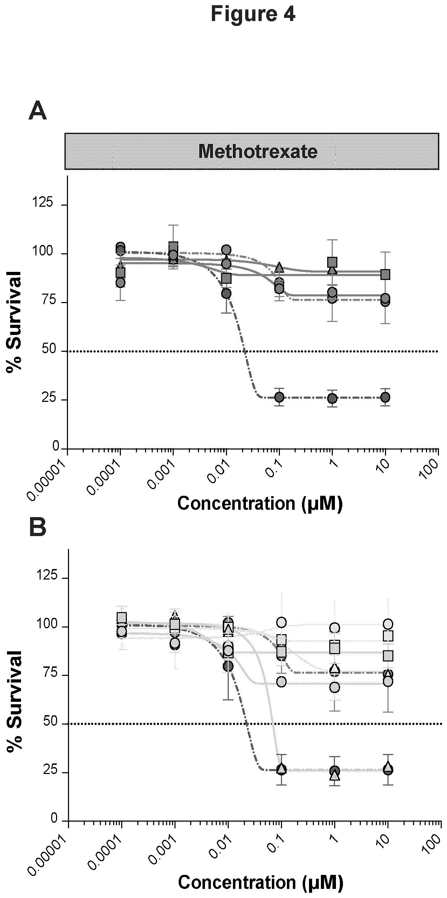

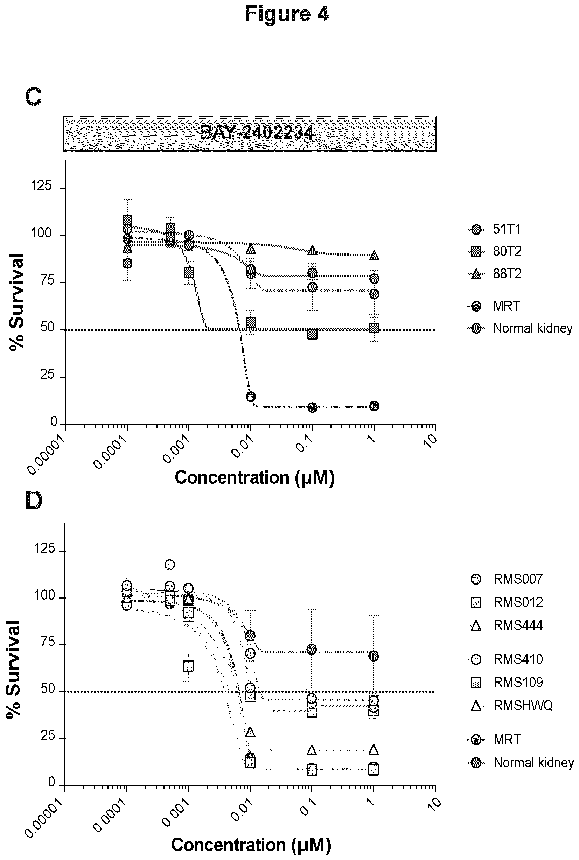

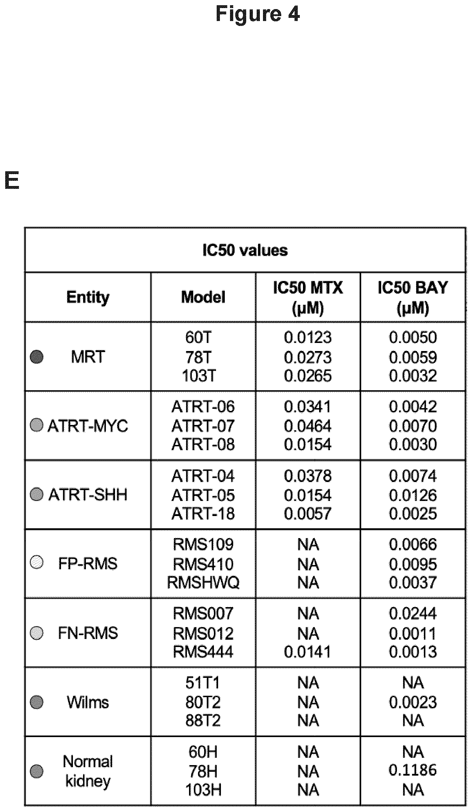

**Figure 3****-** BAY-2402234 delays MRT growth in vivo. A) Experimental overview of in vivo BAY-2402234 testing. Mice were subcutaneously injected with MRT organoids. When tumor volumes reached 200 mm3, mice received oral administration of 4 mg/kg BAY or saline vehicle for a duration of four weeks. B) Tumor growth of MRT model 103T PDX mice (n = 6 mice per treatment arm) treated with either saline vehicle or 4 mg/kg BAY. Data are represented as means ± SEM. P value was calculated using a two-tailed unpaired Student's t test.

## Claims

1. An inhibitor of the human dihydroorotate dehydrogenase (DHODH) enzyme for use in treating cancer in a patient, wherein the cancer is associated with cells in which the functional activity of SMARCB1 is low or absent.

2. The inhibitor according to Claim 1 wherein the patient has, or is suspected of having, a cancer selected from the group consisting of malignant rhabdoid tumors, atypical teratoid/rhabdoid tumors, epithelioid sarcomas, synovial sarcomas, undifferentiated sarcomas with or without rhabdoid features, extra skeletal myxoid chondrosarcomas, renal medullary carcinomas, mucinous carcinomas of the pancreas, malignant peripheral nerve sheath tumors, schwannomas, familial and sporadic schwannomatosis, cribriform neuroepithelial tumors, embryonal central nervous system tumors with or without rhabdoid features, choroid plexus carcinomas, teratoma, primitive neuroectodermal tumors, poorly differentiated chordomas, non-Hodgkin lymphoma, and chronic myeloid leukemia.

3. The inhibitor of DHODH enzyme according to claims 1 and 2, wherein the patient has or is suspected of having malignant rhabdoid tumors (MRT) and/or atypical teratoid/ rhabdoid tumors (AT/RT).

4. The inhibitor according to any one of the preceding claims wherein the patient is assessed prior to commencement of treatment with the inhibitor.

5. The inhibitor according to any one of the preceding claims wherein assessment of the patient comprises providing a sample of cells from a patient and assessing the functional activity of SMARCB1 therein.

6. The inhibitor according to Claim 5 wherein the cells are cancer cells.

7. The inhibitor according to Claim 5 or 6 wherein assessing the functional activity of SMARCB1 comprises measuring the amount of SMARCB1 protein, genomic DNA or cDNA sequence, and/or mRNA encoding the same, in the cells.

8. The inhibitor according to any one of the preceding claims wherein assessment of the patient comprises diagnosing the type of cancer from which the patient is suffering.

9. The inhibitor according to Claim 1 wherein the DHODH inhibitors are selected from the list of BAY-2402234, AG636/AUR-108, Farudodstat, GTX-916, GTX-196, ASLAN003, JNJ-74856665, and PTC299.

10. The inhibitor according to any one of the preceding claims wherein the inhibitor is for administration by a route selected from the group consisting of parenteral, intratumoral, oral, intravenous, transdermal and intramuscular routes.

11. The inhibitor according to any one of the preceding claims wherein the DHODH inhibitor is for administration at a dose of between 4-500 mg/kg per dose.
